# EUROPÄISCHE PATENTSCHRIFT

(11) **EP 2 953 555 B1**
(45) Veröffentlichungstag und Bekanntmachung des Hinweises auf die Patenterteilung: **04.01.2017**
(21) Anmeldenummer: 14706263.2
(22) Anmeldetag: 16.01.2014
(51) Int. Cl.: A61B 17/3209, A61B 17/34

(54) **VORRICHTUNG FÜR EINEN ZUGANG IN VORGEFORMTE KÖRPERHÖHLEN**
DEVICE FOR ACCESS TO PREFORMED BODY CAVITIES
DISPOSITIF PERMETTANT D'ACCÉDER À DES CAVITÉS CORPORELLES PRÉFORMÉES

(30) Priorität: 07.02.2013 AT 500932013
(43) Veröffentlichungstag der Anmeldung: 16.12.2015
(73) Patentinhaber: ACMIT Gmbh, 2700 Wiener Neustadt (AT)
(72) Erfinder: STOCKER, Peter, 8720 Knittelfeld (AT); WIESER, Mario, 8010 Graz (AT)
(74) Vertreter: Wirnsberger, Gernot
(86) Internationale Anmeldenummer: PCT/AT2014/050013
(87) Internationale Veröffentlichungsnummer: WO 2014/121313

(56) Entgegenhaltungen:
- WO-A1-2013/023016
- WO-A2-2004/069035
- WO-A2-2011/135070
- FR-A- 949 943
- US-A1- 2011 196 205

## Beschreibung

Die Erfindung betrifft eine Vorrichtung für einen Zugang in vorgeformte Körperhöhlen wie Thoraxdränagen, insbesondere für die Arthroskopie oder Laparoskopie am menschlichen oder tierischen Körper, umfassend einen Sockel, einen im Sockel geführten Schaft, der länger als der Sockel ausgebildet ist, und ein im Schaft angeordnetes und in diesem führbares Schnittinstrument, das bei Vorwärtsbewegung den Schaft bis zu einer vorbestimmten Position über den Sockel hinaus mitführt.

Arthroskopische Eingriffe am menschlichen oder tierischen Körper zählen heute zu Standardtechniken der operativen Medizin. Insbesondere die Arthroskopie am menschlichen Körper, beispielsweise im Bereich des Knies oder der Schulter, stellt einen Routineeingriff dar. Jährlich werden viele tausende Operationen am menschlichen Knie vorgenommen, beispielsweise bei der Sanierung von Kreuzbandrissen oder Schäden des Meniskus.

Wenngleich die Arthroskopie am menschlichen Knie einen Routineeingriff darstellt, der in der Regel relativ rasch und in vielen Fällen auch nur unter Spinalamnesie oder Plexusamnasie des arthroskopierten Bereiches durchgeführt werden kann, kann es immer wieder zu Komplikationen kommen. Diese Komplikationen betreffen einerseits die Operation als solche. Andererseits kann eine unsachgemäße Operation auch postoperative Syndrome in Form von Blutungen, Infektionen, Wundheilungsstörungen, Funktionsstörungen und Schmerzen auslösen. Darüber hinaus ist insbesondere zu beachten, dass die Einschleppung von Bakterien beispielsweise in die Gelenkshöhle eines menschlichen Gelenkes nach der Operation zu schwerwiegenden Entzündungen des operierten Gelenkes bzw. Hohlraumes führen kann.

Das Dokument US 2011/0196205 A1, auf welchem der Oberbegriff von Anspruch 1 beruht, offenbart eine an einem Körpergewebe befestigbare chirurgische Vorrichtung mit einer Öffnung, in welcher ein Operationswerkzeug geführt werden kann.

Das Dokument FR 949 943 A offenbart eine Vorrichtung für einen Zugang in Körperhöhlen mit einem in einem Schaft führbaren Werkzeug.

Die Arthroskopie am menschlichen Knie wird heute standardmäßig so vorgenommen, dass der Operateur mit einem Skalpell einen Schnitt in die Haut einbringt. Im Anschluss sind die unter der Haut liegenden subkutanen Bereiche bindegewebigen Schichten ebenfalls zu durchtrennen, um später in die Kniegelenkshöhle vordringen zu können. Dabei sind neben einer Fettschicht auch darunterliegende straffe Bindegewebsschichten zu durchtrennen. Insbesondere beim Kniegelenk ist es erforderlich, Fasern des sogenannten Reservestreckapparates teilweise zu durchtrennen, um zum Kniegelenksraum vordringen zu können. Dabei erfolgt eine Durchtrennung der Fasern meist auch quer zur Faserrichtung, sodass diese oftmals geschädigt werden. Ist ein entsprechender Schnitt vorgenommen, wird in der Folge im Bereich des Schnittes ein Rohr mit einer Kamera eingeführt, um eine Gelenksinspektion vornehmen zu können. Hierbei ist es oftmals erforderlich, die entsprechende Extremität umzulagern, um den gesamten Gelenksraum gut inspizieren zu können. Dabei können sich allerdings die Haut und die darunterliegenden Bereiche relativ zueinander verschieben, sodass der ehemals eingebrachte Schnitt in den einzelnen Schichten nicht mehr übereinanderliegt, sondern die einzelnen Schnittbereiche zueinander verschoben sind. In einem solchen Fall ist es erforderlich, neuerlich einen Schnitt einzubringen, was unerwünscht ist. Ist gegebenenfalls nach mehreren Schnitten die Kamera in das Kniegelenk eingeführt und ein allfälliger Schaden am Gelenksapparat festgestellt, wird in einem gesonderten Bereich das Arthroskopieinstrumentarium eingebracht, wobei wiederum eine Inzision durchgeführt werden muss, sodass sich wiederum die gleichen Probleme ergeben, insbesondere bei einer Umlagerung. Ist der festgestellte Schaden saniert, werden das Arthroskopieinstrumentatarium und die Kamera entnommen und die Schnitte mittels Nahtmaterial verschlossen.

Eine Operation wie vorstehend beschrieben dauert beispielsweise für die Behebung eines Meniskusschadens üblicherweise ca. 30 Minuten, wenn keine Komplikationen in der Operationsführung auftreten. Dies bezieht sich allerdings auf einen erfahrenen Operateur. Für einen solchen wäre es noch effizienter, wenn die Operationszeit verkürzt werden könnte. Für einen unerfahrenen Operateur kann die Operationszeit deutlich höher liegen. Insbesondere stellt der schonend hergestellte Zugang zum Knie für einen unerfahrenen Operateur oftmals eine größere Schwierigkeit dar.

Hieran anknüpfend ist es Aufgabe der Erfindung, eine Vorrichtung für die Arthroskopie anzugeben, mit der rasch und in sicherer Weise ein Körperzugang geschaffen werden kann, beispielsweise für die Arthroskopie und Laproskopie oder allgemein eine Eindringung in vorgeformte Körperhöhlen eines menschlichen Gelenkes oder andere vorgeformte Körperhöhlen.

Diese Aufgabe wird gelöst durch eine Vorrichtung der eingangs genannten Art, bei welcher der Schaft Führungsnuten aufweist, in welchen seitliche Fortsätze des Schnittinstrumentes gleiten.

Eine erfindungsgemäße Vorrichtung weist den Vorteil auf, dass der Sockel unmittelbar oder mittelbar am menschlichen Körper befestigt werden kann und anschließend als Stabilisierung für den eingeführten bzw. einzuführenden Schaft dient, in dessen Inneren das Schnittinstrument ruhig führbar ist. Durch die entsprechende Anordnung der einzelnen Komponenten ist zunächst nur die Fixierung der Vorrichtung in einem Bereich des Körpers erforderlich, in welchem operiert werden soll. Wird das Schnittinstrument vorangetrieben, führt dieses den erforderlichen Schnitt mit einer gewünschten Tiefe im menschlichen Körper aus. Gleichzeitig wird bis zu einer vorbestimmten Position auch der Schaft durch das vorangetriebene Schnittinstrument mitgenommen, sodass der Schaft unter gleichzeitiger Umhüllung des Schnittinstrumentes in den menschlichen Körper miteindringt. Dadurch werden zunächst die Haut und dann die daran anschließenden subkutanen Bereiche vom Schnittinstrument durchtrennt und der umliegende Schaft, der vom Schnittinstrument in den Körper hinein mitgenommen wird, spreizt die geschnittene Haut sowie die darauffolgenden Schichten auf, sodass für eine spätere Operation ein perfekter Zugang gegeben ist. Ist das Schnittinstrument mit dem Schaft bis zu einem gewünschten Punkt in den menschlichen Körper eingeführt, kann das Schnittinstrument aus dem Schaft gezogen werden. Der Sockel samt Schaft bleibt am menschlichen Körper befestigt und bildet nunmehr den Zugang für das einzuführende Arthroskopieinstrumentarium. Ist die Operation beendet, wird das Arthroskopieinstrumentarium zunächst aus dem Schaft geführt. Anschließend wird der Schaft aus dem Sockel entnommen und letztlich der Sockel entfernt.

Für eine ruhige Führung des Schnittinstrumentes ist vorgesehen, dass der Schaft Führungen für das Schnittinstrument aufweist, welche mit korrespondierenden Strukturen des Schnittinstrumentes zusammenwirken. Erfindungsgemäß weist der Schaft Führungsnuten auf, in welchen seitliche Fortsätze des grundsätzlich länglich ausgebildeten Schnittinstrumentes gleiten. Auch andere Arten von Führungen sind möglich, welche eine lineare Translation des Schnittinstrumentes im Schaft ermöglichen, ohne dass sich das Schnittinstrument relativ zum Schaft dreht.

Damit das Schnittinstrument den Schaft in den Körper hinein mitnimmt, ist im Schaft bevorzugt zumindest ein Anschlag vorgesehen, an dem das Schnittinstrument nach Einführen in den Schaft und weiterer Vorwärtsbewegung schließlich anschlägt, um den Schaft mitzuführen. Das Schnittinstrument wird dann zunächst in den Schaft eingeführt und gleitet in diesem. Ist der Anschlag erreicht, nimmt das Schnittinstrument den Schaft bei weiterer Vorwärtsbewegung mit. Die Position des Anschlags ist dabei zweckmäßigerweise so gewählt, dass der Schaft mitgenommen wird, sobald das Schnittinstrument in den menschlichen Körper eindringt. Schnittinstrument und Schaft dringen dann quasi gemeinsam in den menschlichen Körper ein, wobei eine Schneidwirkung erzeugende Spitze des Schnittinstrumentes in Vortriebsrichtung knapp vor dem nachfolgenden Schaftende liegt.

Besonders bevorzugt ist vorgesehen, dass das Schnittinstrument an einem ersten Ende mit zumindest einer Schneide ausgebildet ist und im Schaft führbar ist, bis die zumindest eine Schneide über den Schaft vorsteht, vorzugsweise etwa im Bereich eines Schaftendes geringfügig vorsteht. Dies ermöglicht in besonders effizienter Weise eine Kombination von einzubringendem Schnitt und Vortrieb des Schaftes zum Schaffen eines Zugangs für ein Arthroskopieinstrumentarium.

Das Schnittinstrument weist an einem Ende zumindest eine Schneide auf. Es können aber auch mehrere Schneiden vorgesehen sein. Um beispielsweise bei der Arthroskopie eines menschlichen Knies die Fasern des Reservestreckapparates nicht allzu sehr zu verletzen und damit postoperative Symtome zu vermeiden, liegt der Verlauf der einen Schneidkante oder liegen die gegebenenfalls mehreren Schneidkanten in einer einzigen Ebene. Es ist dann möglich, das Schnittinstrument so zu positionieren, dass ein Schnitt parallel zu einer Faserrichtung verläuft, wodurch eine Faserschädigung minimierbar ist. Dabei kann die zumindest eine Schneide konisch und/oder von einem Zentrum zu Seiten des Schnittinstrumentes hin zurückverlaufend ausgebildet sein.

Eine vom schneidaktiven Ende des Schnittinstrumentes zu dessen gegenüberliegendem Ende konisch verbreiternd verlaufende Kontur im Anschluss an die Schneidkante oder die Schneidkanten ermöglicht ein sanftes Aufspreizen des beschnittenen Gebietes, das nachfolgend vom Schaft noch weiter aufgespreizt wird. Wenn die Schneidkante oder die Schneidkanten zu den Seiten des Schnittinstrumentes hin zurückverlaufend ausgebildet sind, also von einem Zentrum des schneidaktiven ersten Endes des Schnittinstrumentes zu dessen gegenüberliegenden zweiten Ende zurücklaufen, ist ein Schnittdruck besonders gering. Dies wirkt sich günstig auf einen Erhalt der Faserstruktur aus. Darüber hinaus erfolgt ein etwas verlangsamtes Schneiden im Vergleich mit gerade verlaufend ausgebildeten Schneidkanten, was ebenso von Vorteil ist. Gleichzeitig sind auch ein gleichmäßiger Schnittdruck und eine besonders gute Zentrierung gegeben.

Der Sockel ist mit Vorteil aus einem elastischen Material gebildet, insbesondere einem elastisch verformbaren Kunststoff. Der Sockel dient als Stabilisierungseinheit für den eingeführten Schaft. Es kann allerdings erforderlich sein, nach Einbringung eines Schnittes und bei bereits erfolgter Positionierung des Schaftes im Körper diesen noch um dessen Längsachse auszulenken. Dies ist möglich, wenn der Sockel aus einem elastischen Material gebildet ist, das eine bestimmte Auslenkung bzw. innerhalb gewisser Grenzen ein Kippen des darin gehaltenen Schaftes ermöglicht.

Der Sockel kann eine oder mehrere Seitenwände aufweisen, die einen Hohlraum definieren, wobei eine erste Öffnung in einem kopfseitigen Bereich und eine zweite Öffnung in einem gegenüberliegenden Bereich des Sockels vorgesehen sind. Der Schaft wird dann im Bereich der ersten Öffnung im kopfseitigen Bereich eingeführt und dort vom Sockel gehalten. Durch die Ausbildung des Sockels aus einem elastischen Kunststoff wird auch die gewünschte Winkelvariabilität für den Schaft erzielt, wenn dieser im Sockel positioniert ist. Hierbei kann im kopfseitigen Bereich des Sockels ein sich um die erste Öffnung erstreckender wulstförmiger Fortsatz angeordnet sein, in dem benachbart zur zweiten Öffnung hin zumindest ein Rücksprung ausgebildet ist und der vorzugsweise transparente Schaft mit einer oder mehreren seitlichen Verankerungsnasen versehen ist, welche in den zumindest einen Rücksprung eingreifen. Die Verankerungsnasen greifen bevorzugt genau dann in die Rücksprünge ein, wenn nach Einführung des Schaftes in den Körper dessen Endposition erreicht ist. Auch hierfür ist es zweckmäßig, dass der Sockel aus einem elastisch verformbaren Material gebildet ist, weil dann durch geringe Kraftaufbringung der wulstförmige Fortsatz zunächst auseinandergedrückt wird, wonach die Verankerungsnasen in dem oder den Rücksprüngen einrasten. Die Verankerungsnasen sind hierfür mit Vorteil keilförmig ausgebildet, damit ein leichtes Aufdrücken des wulstförmigen Fortsatzes erfolgen kann.

Aus ähnlichen Überlegungen, die bereits für das Schnittinstrument ausgeführt wurden, ist auch der Schaft an einem ersten Ende verjüngt zulaufend und am gegenüberliegenden mit einem sich erweiternden offenen Konus ausgebildet. Durch die verjüngt zulaufende Ausbildung am ersten Ende wird eine keilförmige Wirkung beim Einführen des Schaftes in den Körper erzielt, was zu einem sanften Aufspreizen der geschnittenen Bereiche führt. Der sich verbreiternde Konus am gegenüberliegenden Ende ist vorgesehen, um zum einen den Schaft nur bis zu diesem Konus in den Sockel vortreiben zu können. Zum anderen ist die trichterförmige Öffnung am Ende des Schaftes zweckmäßig, um in der Folge das Arthroskopieinstrumentarium treffsicher einführen zu können, was insbesondere für unerfahrene Operateure einen Vorteil darstellt.

Am Schnittinstrument ist zweckmäßigerweise endseitig eine Führungsnadel angeordnet. Die Führungsnadel dient der Stabilisierung des Schnittinstrumentes.

Nicht zwingend, aber mit großem Vorteil ist eine am Körper klebend befestigbare Basis vorgesehen, auf welcher der Sockel befestigt ist. Der Sockel liegt dann nicht unmittelbar am Körper an, sondern auf der Basis, die am Körper angeklebt werden kann. Es ist dann eine besonders einfache Positionierung der Vorrichtung gegeben. Hierfür kann insbesondere vorgesehen sein, dass die Basis selbstklebend ist, sodass die gesamte Vorrichtung ohne weiteres Hilfsmittel auf den menschlichen Körper geklebt werden kann. Dabei ist die Basis vorzugsweise plättchenförmig, also möglichst dünn, ausgebildet und mit dem Sockel über eine Sollbruchstelle verbunden. Der über der Basis, in aller Regel eine Membran, angeordnete Sockel dient dann als Führung für den Schaft und das darin geführte Schnittinstrument, überdeckt jedoch auch die Basis und fungiert somit auch als Keimbarriere. Ist die Operation beendet und der Schaft bereits aus dem Sockel entfernt, kann der Sockel durch die Sollbruchstelle von der Basis bzw. Membran gelöst werden. Die Membran kann dann am menschlichen Körper klebend zurückbleiben, um einen Wundverschluss sicherzustellen. In diesem Fall eignet sich die Vorrichtung nicht nur zur Schaffung eines Zugangs für die Arthroskopie, sondern stellt auch eine optimale Wundversorgung sicher.

Für eine Arthroskopie, die kameraunterstützt ist, empfiehlt sich ein Set aus zwei erfindungsgemäßen Vorrichtungen. Dabei weisen die Schnittinstrumente jeweils eine Führungsnadel auf, wobei die Führungsnadeln unterschiedliche Längen aufweisen. Eine erste Vorrichtung mit der kürzeren Führungsnadel ist dann für das Einbringen eines Schnittes und einen Zugang für eine Kamera vorgesehen. Die zweite Vorrichtung mit der längeren Führungsnadel dient zur Schaffung eines Zugangs für ein einzusetzendes Arthroskopieinstrumentarium.

Weitere Merkmale, Vorteile und Wirkungen der Erfindung ergeben sich aus dem nachfolgend dargestellten Ausführungsbeispiel. In den Zeichnungen, auf welche dabei Bezug genommen wird, zeigen:
Fig. 1 eine Explosionsdarstellung einer erfindungsgemäßen Vorrichtung;
Fig. 2 einen Schnitt durch eine erfindungsgemäße Vorrichtung;
Fig. 3 eine erfindungsgemäße Vorrichtung in einem zusammengebauten und in einem menschlichen Körper eingeführten Zustand.

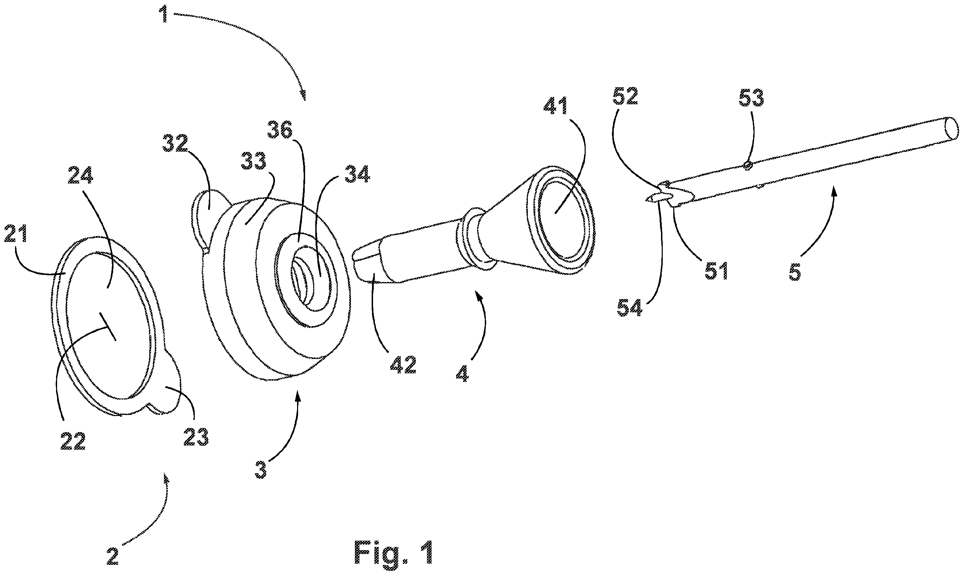

In Fig. 1 sind die einzelnen Teile einer erfindungsgemäßen Vorrichtung 1 in einer Explosionszeichnung dargestellt. Die erfindungsgemäße Vorrichtung 1 weist eine Basis 2, einen Sockel 3, einen Schaft 4 und ein Schnittinstrument 5 auf. Diese Komponenten sowie deren Zusammenwirken sind nachstehend erläutert.

Die Basis 2 ist als leicht durchdringbare Membran ausgebildet, wobei ein zentrales Kunststoffhäutchen 24 vorgesehen ist, das von einem etwas stärkeren Kunststoffring 21 umgeben ist. Die Membran weist des Weiteren einen Schlitz 22 auf, in welchen das später erläuterte Schnittinstrument 5 zunächst eindringt. Die Basis 2 bzw. die Membran ist an einem unteren Ende selbstklebend ausgeführt, sodass die Membran und damit auch die weiteren Komponenten auf einfache Weise an einem menschlichen Körper angebracht werden kann, um später einen Zugang für ein Arthroskopieinstrumentarium zu schaffen. Die Membran dient vor allem dazu, im Bereich eines späteren Schnittes eine Keimbarriere aufzubauen. Darüber hinaus weist die Basis 2 eine Lasche 23 auf, mit welcher die Basis 2 nach der Operation oder gegebenenfalls auch nach einer temporären Wundversorgung wieder leicht vom Körper gelöst werden kann.

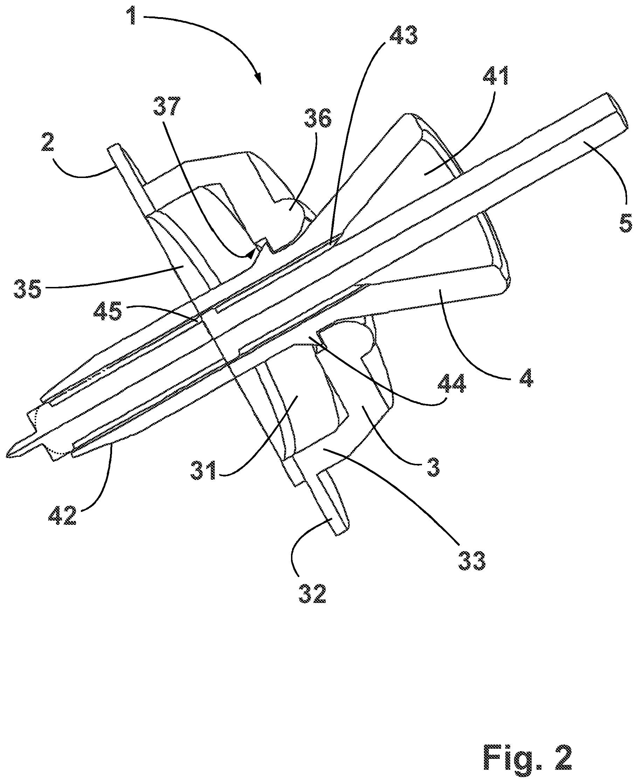

Der Sockel 3, dessen Aufbau in der Schnittdarstellung gemäß Fig. 2 besonders gut ersichtlich ist, ist auf der Basis 2 angeordnet und mit dieser über eine Sollbruchstelle verbunden. Der Sockel 3 weist einen zentralen Hohlraum 31 auf, der durch eine Seitenwand 33 definiert ist. Der Hohlraum 31 kann bei Bedarf mit einer Flüssigkeit gespült werden. Möglich ist es auch, einen Über- oder Unterdruck anzulegen, wenn dies aus operationstechnischen Gründen erforderlich sein sollte. Der Sockel 3 weist eine bestimmte Höhe auf, die jedoch geringer ist als eine Länge des später zu erläuternden Schaftes 4. Der Sockel 3 weist eine erste Öffnung 34 im kopfseitigen bzw. deckseitigen Bereich und eine zweite Öffnung 35 am gegenüberliegenden, dem der Membran benachbarten Ende auf. Die erste Öffnung 34 ist vom Durchmesser her so bemessen, dass der Schaft 4 passgenau aufgenommen werden kann. Die zweite Öffnung 35 ist wesentlich größer ausgebildet und überdeckt die darunterliegende Membran bzw. Basis 2 im Wesentlichen vollständig. Im Bereich des der Membran benachbarten Endes des Schaftes 3 ist eine Lasche 32 vorgesehen. Am gegenüberliegenden Ende des Sockels 3 liegt ein wulstförmiger Fortsatz 36 vor, der letztlich die erste Öffnung 34 definiert. Wie insbesondere in Fig. 2 ersichtlich, ist der wulstförmige Fortsatz 36 kopfseitig leicht gerundet. Zum Hohlraum 31 hin ist ein umlaufender Rücksprung 37 vorgesehen, wenngleich grundsätzlich auch mehrere solcher Rücksprünge 37 vorhanden sein können. Zweckmäßig ist es jedoch, dass ein einziger Rücksprung 37 vorgesehen ist, der wie der Schaft 3 rund ausgebildet ist und um den Öffnungsbereich im Bereich der ersten Öffnung 34 rundherumläuft.

Der bereits erwähnte Schaft 4 ist länglich und transparent ausgebildet. Eine Länge des Schaftes 4 ist wesentlich größer als eine Höhe des Sockels 3. Zweckmäßig ist es, dass der Schaft 4 zumindest zweimal so lang ist wie die Höhe des Sockels 3. Der Schaft 4 weist ein erstes, in den Körper einführbares Ende auf, das konisch verbreiternd bzw. mit einem sich in Richtung eines gegenüberliegenden Endes des Schaftes 4 verlaufenden endseitigen Konus 42 ausgebildet ist. Dadurch spreizt der Schaft 4 beim Eindringen in den menschlichen Körper zunächst sanft die Haut und dann das darunterliegende Gewebe auf. Im Anschluss erstreckt sich der Schaft 4, der hohl ausgebildet ist, im Wesentlichen zylindrisch, wobei an einer Außenseite keilförmige Verankerungsnasen 44 vorgesehen sind. Im Anschluss an die Verankerungsnasen 44 ist ein offener Konus 41 vorgesehen, an welchem sich der Schaft 4 unter Bildung eines trichterförmigen Einlasses für weitere Instrumente verbreitert. Optional können im Schaft 4 innen zwei weitere, nicht dargestellte dünne Membranen vorgesehen sein, die vom Schnittinstrument 5 bei Vorwärtsbewegung bzw. Einführen zu durchtrennen sind. Diese zusätzlichen Membranen im Schaft 4 dienen als zusätzliche Keim- und Flüssigkeitsbarrieren. Im Inneren sind des Weiteren innenseitige Nuten 43 und zumindest ein Anschlag 45 vorgesehen.

Das Schnittinstrument 5 schließlich ist zylinderförmig ausgeführt und kann hohl oder auch massiv ausgebildet sein. Das Schnittinstrument 5 weist an einem Ende 51 eine Führungsnadel 54 mit einer endseitigen Spitze auf. Im Anschluss an die Führungsnadel 54 erstrecken sich Schneiden 52, die wie dargestellt gerade verlaufen können, bevorzugt jedoch zur Seite des Schnittinstrumentes 5 hin zurückverlaufend ausgebildet sind, wie dies in Fig. 2 strichliert angedeutet ist, sodass unter anderem ein geringer Schnittdruck bei stabiler Führung des Schnittinstrumentes 5 erreicht wird und eine Faserbeschädigung möglichst vermeidbar ist. Das Schnittinstrument 5 ist wie der Schaft 4, mit dem es zusammenwirkt, im Wesentlichen länglich ausgebildet, weist noch an einer Seite positionierte Fortsätze 53 auf, die mit dem Anschlag 45 des Schaftes 4 zusammenwirken. Die Fortsätze 53 sind geringfügig höher als die Breite der Schneiden 52 ausgebildet, sodass die Schneiden 52 am Anschlag 45 des Schaftes 4 vorbeigleiten können. Möglich ist es auch, dass die Fortsätze 53 in einer um 90° verdrehten Position am Schnittinstrument 5 angeordnet sind und in anderen Nuten als die Schneiden 52 gleiten.

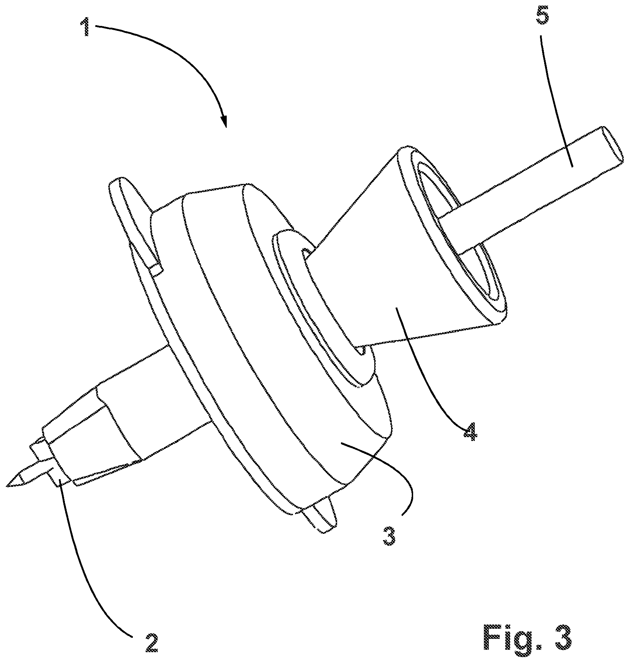

Unter Bezugnahme auf Fig. 1 bis 3 wird die Funktionsweise einer erfindungsgemäßen Vorrichtung 1 bei einer Arthroskopie erläutert. Die Vorrichtung 1 liegt bei der Operation bereits als zusammengebautes Instrumentarium vor. Dabei ist der Sockel 3 auf der Basis 2 befestigt und mit dieser über die Sollbruchstelle verbunden. Der Schaft 4 ist bereits im Sockel 3 gehalten, liegt allerdings erst an der Basis 2 bzw. Membran an. Ebenso ist das Schnittinstrument 5 bereits im Schaft 4 positioniert. Wird nun das Schnittinstrument 5 entlang dessen Längsachse vorangetrieben, bewegt sich dieses zunächst aufgrund der Führung durch den Schaft 4 bzw. der Fortsätze 53 in den innenseitigen Nuten 43 des Schaftes 4 ruhig geführt vorwärts. Schließlich erreicht das Schnittinstrument 5 die Membran und durchdringt diese im Bereich des Schlitzes 22. Kurz danach schlagen die Fortsätze 53 des Schnittinstrumentes 5 an den Anschlägen 45 des Schaftes 4 an, sodass eine weitere Vorwärtsbewegung des Schnittinstrumentes 5 eine Mitnahme bzw. Mitführung des Schaftes 4 zur Folge hat. Dies ist dann der Fall, wenn die Schneiden 52 des Schnittinstrumentes 5 den endseitigen Konus 42 des Schaftes 4 geringfügig überragen. Damit der Schaft 4 leicht in die Haut eingleiten kann, sind die Schneiden 52 hierfür etwa so breit ausgebildet wie ein Ende des Schaftes 4. Anschließend wird das Schnittinstrument 5 samt Schaft 4 in den Körper vorangetrieben, bis die in Fig. 2 bzw. 3 dargestellte Position annähernd erreicht ist. Es liegen nun die Verankerungsnasen 44 des Schaftes 4 am wulstförmigen Fortsatz 36 des Sockels 3 an. Da der Sockel 3 aus einem elastischen Material gefertigt ist, lässt sich durch geringe Kraftaufbringung dessen Widerstand überwinden, sodass die Verankerungsnasen 44 über den wulstförmigen Fortsatz 36 gleiten und schließlich in den Rücksprüngen 37 des Sockels 3 zu liegen kommen. Dadurch ist der Schaft 4 während der weiteren Operation perfekt gegen ein unbeabsichtigtes Lösen vom Sockel 3 gesichert. Die elastische Verformbarkeit des Sockels 3 erlaubt es im Übrigen auch während der Operation, den Schaft 4 winkelmäßig auszulenken. Gleichzeitig mit der Fixierung der Verankerungsnasen 44 schlägt der offene endseitige Konus 42 am wulstförmigen Fortsatz 36 an, sodass der Schaft 4 grundsätzlich in beide Richtungen gegen ein weiteres Verschieben gesichert ist. Das Schnittinstrument 5 kann auch nicht weiter bewegt werden. Es ist nun die in Fig. 2 bzw. 3 dargestellte Situation erreicht. Nun wird das Schnittinstrument 5 aus dem Schaft 4 gezogen. Es ist somit ein Zugang in den Körper geschaffen, der an die Basis 2 bzw. Membran anschließt. Es kann nun eine Kamera oder gegebenenfalls ein Arthroskopieinstrument eingeführt werden. Sofern beide Komponenten während der Operation erforderlich sind, also sowohl Kamera als auch das Arthroskopieinstrument, wird wie vorstehend beschrieben an zwei verschiedenen Stellen vorgegangen. Nach der Arthroskopie wird das Arthroskopieinstrument oder auch die Kamera aus dem Schaft 4 gezogen. Danach wird der Schaft 4 vom Sockel 3 gelöst, indem dieser gegen die Kraft der Verankerung durch die Verankerungsnasen 44 in Kombination mit dem Rücksprung 37 gezogen wird. Aufgrund der elastischen Verformbarkeit des Sockels 3 ist dies mit einem moderaten Kraftaufwand möglich. Nunmehr befindet sich lediglich der Sockel 3 auf der Membran. Zum Abziehen des Sockels 3 von der Membran wird die Lasche 32 verwendet. Aufgrund der Verbindung über eine Sollbruchstelle kann der Sockel 3 von der Membran gelöst werden. Die Basis 2 bzw. Membran kann ebenfalls über deren Lasche 23 entfernt werden. Von Vorteil ist es jedoch, wenn die Membran zur Versorgung des Schnittes bzw. der Wunde noch bis zur vollständigen Wundheilung am Körper bleibt. Da die Membran nur einen kleinen Schlitz aufweist und in dem Zentralbereich ebenfalls elastisch ausgebildet ist, schließt sich diese quasi über der Wunde, was eine sehr gute Heilung ermöglicht und vor allem die offene Wunde vor Keimen schützt.

## Patentansprüche

1. Vorrichtung (1) für einen Zugang in vorgeformte Körperhöhlen wie Thoraxdränagen, insbesondere für die Arthroskopie oder Laparoskopie am menschlichen oder tierischen Körper, umfassend einen Sockel (3), einen im Sockel (3) geführten Schaft (4), der länger als der Sockel (3) ausgebildet ist, und ein im Schaft (4) angeordnetes und in diesem führbares Schnittinstrument (5), das bei Vorwärtsbewegung den Schaft (4) bis zu einer vorbestimmten Position über den Sockel (3) hinaus mitführt, **dadurch gekennzeichnet, dass** der Schaft (4) Führungsnuten (43) aufweist, in welchen seitliche Fortsätze (53) des Schnittinstrumentes (5) gleiten.

2. Vorrichtung (1) nach Anspruch 1, wobei im Schaft (4) zumindest ein Anschlag (45) vorgesehen ist, an dem das Schnittinstrument (5) nach Einführen in den Schaft (4) und Vorwärtsbewegung anschlägt, um den Schaft (4) mitzuführen.

3. Vorrichtung (1) nach einem der Ansprüche 1 oder 2, wobei das Schnittinstrument (5) an einem ersten Ende (51) mit zumindest einer Schneide (52) ausgebildet ist und im Schaft (4) führbar ist, bis die zumindest eine Schneide (52) über den Schaft (4) vorsteht, vorzugsweise etwa im Bereich eines Schaftendes (46) geringfügig vorsteht.

4. Vorrichtung (1) nach Anspruch 3, wobei die zumindest eine Schneide (52) konisch und/oder von einem Zentrum zu Seiten des Schnittinstrumentes (5) hin zurückverlaufend ausgebildet ist.

5. Vorrichtung (1) nach einem der Ansprüche 1 bis 4, wobei der Sockel (3) aus einem elastischen Material gebildet ist, insbesondere einem elastisch verformbaren Kunststoff.

6. Vorrichtung (1) nach einem der Ansprüche 1 bis 5, wobei der Sockel (3) eine oder mehrere Seitenwände (33) aufweist, die einen Hohlraum (31) definieren, und eine erste Öffnung (34) in einem kopfseitigen Bereich und eine zweite Öffnung (35) in einem gegenüberliegenden Bereich des Sockels (3) vorgesehen sind.

7. Vorrichtung (1) nach einem der Ansprüche 1 bis 6, wobei im kopfseitigen Bereich des Sockels (3) ein sich um die erste Öffnung (34) erstreckender wulstförmiger Fortsatz (36) angeordnet ist, in dem benachbart zur zweiten Öffnung (35) hin zumindest ein Rücksprung (37) ausgebildet ist und der Schaft (4) mit einer oder mehreren seitlichen Verankerungsnasen (44) versehen ist, welche in den zumindest einen Rücksprung (37) eingreifen.

8. Vorrichtung (1) nach einem der Ansprüche 1 bis 7, wobei der Schaft (4) an einem ersten Ende verjüngt zulaufend und am gegenüberliegenden Ende mit einem sich erweiternden offenen Konus (41) ausgebildet ist.

9. Vorrichtung (1) nach einem der Ansprüche 1 bis 8, wobei am Schnittinstrument (5) endseitig eine Führungsnadel (54) angeordnet ist.

10. Vorrichtung (1) nach einem der Ansprüche 1 bis 9, wobei eine am Körper klebend befestigbare Basis (2) vorgesehen ist, auf welcher der Sockel (3) befestigt ist.

11. Vorrichtung (1) nach Anspruch 10, wobei die Basis (2) selbstklebend ist.

12. Vorrichtung (1) nach Anspruch 10 oder 11, wobei die Basis (2) plättchenförmig ausgebildet ist und mit dem Sockel (3) über eine Sollbruchstelle verbunden ist.

13. Set aus mehreren Vorrichtungen (1) gemäß einem der Ansprüche 1 bis 12, wobei die Schnittinstrumente (5) jeweils eine Führungsnadel (54) aufweisen, wobei die Führungsnadeln (54) unterschiedliche Längen aufweisen.

## Claims

1. Device (1) for access to preformed body cavities, such as thoracic drains, in particular for arthroscopy or laparoscopy on a human or animal body, comprising a base (3), a shaft (4) guided in the base (3), which shaft is embodied longer than the base (3), and a cutting instrument (5) which is arranged in the shaft (4) and is guidable therein and which, upon forward movement, carries the shaft (4) as far as a predetermined position beyond the base (3), **characterised in that** the shaft (4) comprises guide grooves (43) in which lateral extensions (53) of the cutting instrument (5) slide.

2. Device (1) according to claim 1, wherein at least one stop (45) is provided in the shaft (4), against which stop the cutting instrument (5) bears to carry the shaft (4) along after being inserted in the shaft (4) and moved forward.

3. Device (1) according to claim 1 or 2, wherein the cutting instrument (5) is embodied at a first end (51) with at least one blade (52) and is guidable in the shaft (4) until the at least one blade (52) protrudes past the shaft (4), preferably slightly protruding approximately in the region of a shaft end (46).

4. Device (1) according to claim 3, wherein the at least one blade (52) is embodied conically and/or in a receding manner from a centre to sides of the cutting instrument (5).

5. Device (1) according to any one of claims 1 to 4, wherein the base (3) is made from an elastic material, in particular an elastically deformable plastic.

6. Device (1) according to any one of claims 1 to 5, wherein the base (3) includes one or more side walls (33), which define a hollow space (31), and a first opening (34) in a head region and a second opening (35) in an opposite region of the base (3) are provided.

7. Device (1) according to any one of claims 1 to 6, wherein a bead-shaped extension (36) extending around the first opening (34) is arranged in the head region of the base (3), in which extension at least one recess (37) is embodied adjacent to the second opening (35), and the shaft (4) is furnished with one or more lateral anchoring lugs (44) which engage in the at least one recess (37).

8. Device (1) according to any one of claims 1 to 7, wherein the shaft (4) is embodied in a tapered manner at the first end and with a flared open cone (41) at the opposite end.

9. Device (1) according to any one of claims 1 to 8, wherein a guide pin (54) is arranged (5) on the end of the cutting instrument.

10. Device (1) according to any one of claims 1 to 9, wherein a substrate (2) adhesively attachable to the body is provided, to which substrate the base (3) is attached.

11. Device (1) according to claim 10, wherein the substrate (2) is self-adhesive.

12. Device (1) according to claim 10 or 11, wherein the substrate (2) is embodied in the shape of a small plate and is connected to the base (3) by a predetermined breaking point.

13. Set of multiple devices (1) according to any one of claims 1 to 12, wherein the cutting instruments (5) each include a guide pin (54), wherein the guide pins (54) are of different lengths.

## Revendications

1. Dispositif (1) d'accès à des cavités corporelles préformées comme des drainages thoraciques, en particulier pour l'arthroscopie ou la laparoscopie sur le corps humain ou animal, comprenant un socle (3), une tige (4) guidée dans le socle (3) et qui est plus longue que le socle (3) et un instrument de coupe (5) disposé dans la tige (4) et pouvant être guidé dans celle-ci et qui, en cas de mouvement vers l'avant de la tige (4), l'entraîne jusqu'à une position prédéfinie au-delà du socle (3), **caractérisé en ce que** la tige (4) présente des rainures de guidage (43) dans lesquelles glissent des prolongements latéraux (53) de l'instrument de coupe (5).

2. Dispositif (1) selon la revendication 1, dans lequel il est prévu dans la tige (4) au moins une butée (45) contre laquelle l'instrument de coupe (5) bute après l'introduction dans la tige (4) et le mouvement vers l'avant afin d'entraîner la tige (4).

3. Dispositif (1) selon une des revendications 1 ou 2, dans lequel l'instrument de coupe (5) est conformé à une extrémité (51) avec au moins une lame (52) et peut être guidé dans la tige (4) jusqu'à ce que l'au moins une lame (52) dépasse de la tige (4), de préférence dépasse légèrement approximativement au niveau d'une extrémité de la tige (46).

4. Dispositif (1) selon la revendication 3, dans lequel l'au moins une lame (52) a une conformation conique et/ou en retrait depuis un centre vers les côtés de l'instrument de coupe (5).

5. Dispositif (1) selon une des revendications 1 à 4, dans lequel le socle (3) est composé d'un matériau élastique, en particulier d'une matière plastique élastiquement déformable.

6. Dispositif (1) selon une des revendications 1 à 5, dans lequel le socle (3) présente une ou plusieurs parois latérales (33) qui définissent une cavité (31) et qu'il est prévu une première ouverture (34) dans une zone sommitale et une seconde ouverture (35) dans une zone opposée du socle (3).

7. Dispositif (1) selon une des revendications 1 à 6, dans lequel, dans la zone sommitale du socle (3), est disposé un prolongement (36) s'étendant autour de la première ouverture (34) et dans lequel, à proximité et en direction de la seconde ouverture (35), est réalisé au moins un retrait (37) et que la tige (4) est pourvue d'un ou plusieurs becs d'ancrage latéraux (44) qui s'engrènent dans l'au moins un retrait (37).

8. Dispositif (1) selon une des revendications 1 à 7, dans lequel la tige (4) est conformée avec un rétrécissement à la première extrémité et avec un cône ouvert (41) s'élargissant à l'extrémité opposée.

9. Dispositif (1) selon une des revendications 1 à 8, dans lequel, à l'extrémité de l'instrument de coupe (5), une aiguille de guidage (54) est disposée.

10. Dispositif (1) selon une des revendications 1 à 9, dans lequel il est prévu une base (2) pouvant être fixée au corps par collage et sur laquelle le socle (3) peut être fixé.

11. Dispositif (1) selon la revendication 10, dans lequel la base (2) est autocollante.

12. Dispositif (1) selon la revendication 10 ou 11, dans lequel la base (2) est réalisée en forme de plaquette (3) et est reliée au socle par un point de rupture théorique.

13. Ensemble de plusieurs dispositifs (1) selon une des revendications 1 à 12, dans lequel les instruments de coupe (5) présentent respectivement une aiguille de guidage (54), les aiguilles de guidage (54) ayant des longueurs différentes.
